# EUROPEAN PATENT APPLICATION

(11) **EP 1 992 605 A1**
(43) Date of publication of application: **19.11.2008**
(21) Application number: 07737658.0
(22) Date of filing: 02.03.2007
(51) Int. Cl.: C07C 41/18, B01J 31/28, C07C 43/164, C07C 43/18, C07C 269/06, C07C 271/22, C07H 15/18, C07J 9/00, C07B 61/00

(54) **METHOD OF DEUTERIZING BENZYL-POSITION IN -O-BENZYL GROUP**

(30) Priority: 03.03.2006 JP 2006058201
(71) Applicant: Wako Pure Chemical Industries, Ltd., Osaka-shi, Osaka 540-8605 (JP)
(72) Inventor: SAJIKI, Hironao, Gifu-shi Gifu 502-8585 (JP); MAEGAWA, Tomohiro, Gifu-shi Gifu 502-8585 (JP); KURITA, Takanori, Gifu-shi Gifu 502-8585 (JP)
(74) Representative: Winter, Brandl, Fürniss, Hübner Röss, Kaiser, Polte Partnerschaft Patent- und Rechtsanwaltskanzlei
(86) International application number: PCT/JP2007/054010
(87) International publication number: WO 2007/100080

(57) **Abstract**

(PROBLEM)

To provide a method for efficiently and industrially deuterating the benzyl position of a -O-benzyl group formed by introducing a benzyl group, a benzyloxymethyl group and the like as a protecting group.

(SOLUTION)

The deuteration method for the benzyl position of a -O-benzyl group of a compound having the -O-benzyl group which may have a substituent, comprising reacting the compound with a heavy hydrogen source in the coexistence of a palladium/carbon-ethylenediamine complex and hydrogen.

## Description

### TECHNICAL FIELD

The present invention relates to a method for deuterating the benzyl position of a -O-benzyl group of a compound having the -O-benzyl group.

### BACKGROUND ART

A deuterated (deuterated and tritiated) compound is useful for various purposes. A deuterated compound, for example, is very useful in investigating reaction mechanism, substance metabolism and the like and used broadly as a labeled compound. It is further, said that the compound is also useful as a medicine, an agrichemical, an organic EL material and the like because the stability and properties of the compound itself vary according to its isotope effect. It is also said that a tritiated compound is useful as a labeled compound in animal experiments to investigate absorption, distribution, blood concentration, excretion, metabolism and the like of medicines. By these reasons, researches on a deuterated (deuterated and tritiated) compound have been also increasing in these fields using.

A benzyl group, on the other hand, is widely used as, for example, a protecting group for a hydroxyl group of a sugar compound. As selective deuteration of the benzyl position of a -Q-benzyl group that protects a hydroxyl group with the benzyl group would be extremely useful in broad areas of not only synthesis of a labeled compound but also investigation of reaction mechanism, it has been desired to develop a method for selectively deuterating the benzyl position of a -O-benzyl group.

The present inventors reported that a benzyl position was selectively deuterated and labeled in deuterated water in the presence of a small amount of hydrogen gas under ordinary temperature and pressure using palladium/carbon (Pd/C) as a catalyst (non-patent literature 1). However, when the above reaction was used for deuteration of the benzyl position of the -O-benzyl group using a compound (compound having a -O-benzyl group) to which a -O-benzyl group as a reducible functional group was introduced, the objective deuterated compound could not be efficiently obtained due to hydrogenolysis of the -O-benzyl group caused by high hydrogenation activity of the Pd/C catalyst.

The present inventors also developed a palladium/carbon-ethylenediamine complex [Pd/C (en)] having selectivity for a functional group and reported that the this catalyst could be used as a catalyst for selectively hydrogenating the other reducible functional groups such as an olefin, a nitro group and bromine without reducing, for example, the -O-benzyl group of a compound having its hydroxyl group protected with a benzyl group (non-patent literature 2).

Under such circumstances, it is now desired to develop a method for efficiently deuterating the benzyl position of a -O-benzyl group of a compound having the -O-benzyl group.

[non-patent literature 1] Synlett 2002, 1149 - 1151
[non-patent literature 2] J. Org. Chem. 1998, 63, 7990 - 7992

### DISCLOSURE OF INVENTION

### PROBLEM TO BE SOLVED BY THE INVENTION

Considering the above circumstances, the present invention provides a method for deuterating efficiently and industrially the benzyl position of a -O-benzyl group of a compound, to which a group such as a benzyl group and benzyloxymethyl group is introduced as a protecting group for its hydroxyl group, having the -O-benzyl group.

### MEANS FOR SOLVING THE PROBLEM

The present invention relates to an invention of a method for deuterating the benzyl position of a -O- benzyl group of a compound, comprising reacting the compound having the -O- benzyl group which may have a substituent with a heavy hydrogen source in the coexistence of a palladium/carbon-ethylenediamine complex and hydrogen.

### EFFECTS OF THE INVENTION

The method of the present invention for deuterating the benzyl position of a -O-benzyl group in a compound to which the -O-benzyl group as a reducible functional group does not cause any problem associated with a conventional method using Pd/C as a catalyst, that is, the problem that high hydrogenation activity of the Pd/C catalyst causes hydrogenolysis of the -O-benzyl group along with deuteration, and thus can efficiently provide an objective deuterated compound by selectively deuterating the benzyl position of the -O-benzyl group.

### BEST MODE FOR CARRYING OUT THE INVENTION

In the present invention, a heavy hydrogen atom means a deuterium (D) atom or a tritium (T) atom, and deuteration means deuteration and tritiation. A deuteration ratio in the present description means a ratio of the hydrogen atoms substituted with heavy hydrogen atoms to the total hydrogen atoms of the benzyl position in a compound.

In the deuteration method of the present invention, the compound having a -O-benzyl group which may have a substituent is obtained by protecting a hydroxyl group of a compound having the hydroxyl group with a group represented by the general formula [1]:

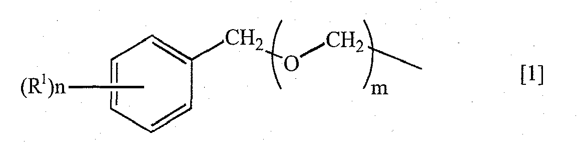

(wherein, n R¹s each independently indicate a light hydrogen atom, a heavy hydrogen atom, an alkyl group or an alkoxy group; n indicates 0 or an integer of 1 to 3; and m indicates 0 or 1), and includes, for example, a compound having a group represented by the general formula [2]:

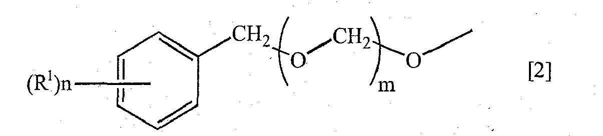

(wherein, R¹, n and m are each the same as above defined), and specifically, for example, a compound represented by general formula [3]:

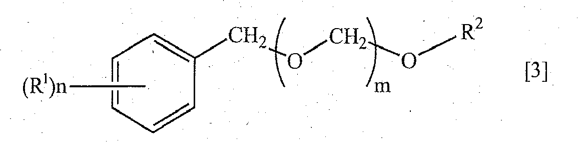

(wherein, R² is an alkyl group, aryl group, aralkyl group or heterocyclic group, which may have a substituent, or a monovalent group derived from a compound having a hydroxyl group, of which the hydrogen atom of the hydroxyl group is substituted with a binding arm; n indicates 0 or an integer of 1 to 3; and m indicates 0 or 1).

The alkyl group indicated by R¹ in the general formulae [1] to [3] may be any of a straight-chained, branched and cyclic group, and includes a group of usually 1 to 3 carbon atoms and preferably of 1 carbon atom, and specifically, for example, a methyl group, an ethyl group, a n-propyl group, an isopropyl group and a cyclopropyl group.

The alkoxy group indicated by R¹ may be a straight-chained or branched group and includes a group of usually 1 to 3 carbon atoms and preferably of 1 carbon atom, and specifically, for example, a methoxy group, an ethoxy group, a n-propoxy group and an isopropoxy group.

n indicates usually 0 or an integer of 1 to 3 and preferably 0 or 1.
m indicates usually 0 or 1.

A typical example of a -O-benzyl group (preferably, a group represented by the general formula [1]) which may have a substituent includes benzyloxy groups such as a benzyloxy group, a p-methylbenzyloxy group, an o-methoxybenzyloxy group, a m-methoxybenzyloxy group, a p-methoxybenzyloxy group, a 3,4-dimethoxybenzyloxy group, a 3,5-dimethoxybenzyloxy group, a 2,3-dimethoxybenzyloxy group, a 2,5-dimethoxybenzyloxy group, a 2,6-dimethoxybenzyloxy group, a 2,3,4-trimethoxybenzyloxy group and a 3,4,5-trimethoxybenzyloxy group; benzyloxymethyloxy groups such as a benzyloxymethyloxy group, a p-methoxybenzyloxymethyloxy group and a 3,4-dimethoxybenzyloxymethyloxy group; and among these, preferably benzyloxy groups such as a benzyloxy group, a p-methoxybenzyloxy group and a 3,4-dimethoxybenzyloxy group; and benzyloxymethyloxy groups such as a benzyloxymethyloxy group, a p-methoxybenzyloxymethyloxy group; and more preferably a benzyloxy group, a p-methoxybenzyloxy group or a benzyloxymethyloxy group.

In general formula [3], the alkyl group indicated by R² which may have a substituent may be any of a straight-chained, branched and cyclic group, and includes a group of usually 1 to 30 carbon atoms and specifically, for example, a methyl group, an ethyl group, a n-propyl group, an isopropyl group, a n-butyl group, an isobutyl group, a sec-butyl group, a tert-butyl group, a n-pentyl group, an isopentyl group, a sec-pentyl group, a tert-pentyl group, a neopentyl group, a 1-methylpentyl group, a n-hexyl group, an isohexyl group, a sec-cyclohexyl group, a tert-cyclohexyl group, a neocyclohexyl group, a n-heptyl group, an isoheptyl group, a sec-heptyl group, a tert-heptyl group, a neoheptyl group, a n-octyl group, an isooctyl group, a sec-octyl group, a tert-octyl group, a neooctyl group, a n-nonyl group, an isononyl group, a sec-nonyl group, a tert-nonyl group, a neononyl group, a n-decyl group, an isodecyl group, a sec-decyl group, a tert-decyl group, a neodecyl group, a n-undecyl group, an isoundecyl group, a sec-undecyl group, a tert-undecyl group, a neoundecyl group, a n-dodecyl group, an isododecyl group, a sec-dodecyl group, a tert-dodecyl group, a neododecyl group, a n-tridecyl group, an isotridecyl group, a sec-tridecyl group, a tert-tridecyl group, a neotridecyl group, a n-tetradecyl group, an isotetradecyl group, a sec-tetradecyl group, a tert-tetradecyl group, a neotetradecyl group, a n-pentadecyl group, an isopentadecyl group, a sec-pentadecyl group, a tert-pentadecyl group, a neopentadecyl group, a n-hexadecyl group, an isohexadecyl group, a sec-hexadecyl group, a tert-hexadecyl group, a neohexadecyl group, a n-heptadecyl group, an isoheptadecyl group, a sec-heptadecyl group, a tert-heptadecyl group, a neoheptadecyl group, a n-octadecyl group, an isooctadecyl group, a sec-octadecyl group, a tert-octadecyl group, a neooctadecyl group, a n-nonadecyl group, an isononadecyl group, a sec-nonadecyl group, a tert-nonadecyl group, a neononadecyl group, a n-icosyl group, an isoicosyl group, a sec-icosyl group, a tert-icosyl group, a neoicosyl group, a n-henicosyl group, an isohenicosyl group, a sec-henicosyl group, a tert-henicosyl group, a neohenicosyl group, a n-docosyl group, an isodocosyl group, a sec-docosyl group, a tert-docosyl group, a neodocosyl group, a n-tricosyl group, an isotricosyl group, a sec-tricosyl group, a tert-tricosyl group, a neotricosyl group, a n-tetracosyl group, an isotetracosyl group, a sec-tetracosyl group, a tert-tetracosyl group, a neotetracosyl group, a n-pentacosyl group, an isopentacosyl group, a sec-pentacosyl group, a tert-pentacosyl group, a neoentacosyl group, a n-hexacosyl group, an isohexacosyl group, a sec-hexacosyl group, a tert-hexacosyl group, a neohexacosyl group, a n-heptacosyl group, an isoheptacosyl group, a sec-heptacosyl group, a tert-heptacosyl group, a neoheptacosyl group, a n-octacosyl group, an isooctacosyl group, a sec-octacosyl group, a tert-octacosyl group, a neooctacosyl group, a n-nonacosyl group, an isononacosyl group, a sec-nonacosyl group, a tert-nonacosyl group, a neononacosyl group, a n-triacontyl group, an isotriacontyl group, a sec-triacontyl group, a tert-triacontyl group, a neotriacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotridecyl group, a cyclotetradecyl group, a cyclopentadecyl group, a cyclohexadecyl group, a cycloheptadecyl group, a cyclooctadecyl group, a cyclononadecyl group, a cycloicosyl group and the like.

The aryl group of the aryl group which may have a substituent, indicated by R² includes a group of usually 6 to 14 carbon atoms and specifically, for example, a phenyl group, a tolyl group, a xylyl group, a naphthyl group, an anthryl group and the like.

The aralkyl group of the aralkyl group which may have a substituent, indicated by R² includes a group of usually 7 to 15 carbon atoms and specifically, for example, a benzyl group, a phenethyl group, a 1-phenylethyl group, a 2-phenylpropyl group, a 3-phenylpropyl group, a phenylbutyl group, a 1-methyl-3-phenylpropyl group, a naphthylmethyl group, a naphthylethyl group, a naphthylpropyl group, a naphthylbutyl group and the like.

The heterocyclic group of the heterocyclic group which may have a substituent, indicated by R² includes preferably, for example, a five-membered ring or six-membered ring having 1 to 3 atoms of usually a nitrogen atom, an oxygen atom or a sulfur atom as a heterogeneous atom, preferably a nitrogen atom or an oxygen atom, and specifically, an aliphatic heterocyclic group such as a pyranyl group, an imidazolyl group, a pyrazolyl group, a pyrazinyl group, a pyrimidinyl group, a pyridazinyl group, a pyrrolidinyl group, a pyrrolinyl group, an imidazolinyl group, a pyrazolidinyl group, a pyrazolinyl group, a piperazinyl group, a morpholinyl group, a quinuclidinyl group, a pyrrolidyl-2-one group, a piperidyl group, a piperidino group, a piperazinyl group and a morpholino group; an aromatic heterocyclic group such as a furyl group, a pyrrolyl group, an indolyl group, a purinyl group, a quinolyl group, a pyridyl group, a pyrazyl group, a pyrimidyl group and an oxazolyl group; and the like.

The substituent of the alkyl group, aryl group, aralkyl group and heterocyclic group which may have a substituent, indicated by R² includes, for example, a lower alkoxy group, an amino group, a hydroxyl group, a carboxyl group and the like.

The lower alkoxy group exemplified as a substituent may be any of a straight-chained, branched or cyclic group and includes usually a group having 1 to 3 carbon atoms and specifically, for example, a methoxy group, an ethoxy group, an n-propoxy group, an isopropoxy group and the like.

The compound having a hydroxyl group of a monovalent group derived from a compound having a hydroxyl group, indicated by R², of which the hydrogen atom of the hydroxyl group is substituted with a bonding arm, is not particularly limited as long as it has a hydroxyl group, and includes typically, for example, alcohols, a compound having a sugar structure, a steroid having a hydroxyl group, an alkaloid having a hydroxyl group, an amino acid having a hydroxyl group, a peptide containing an amino acid residue having a hydroxyl group, terpenes having a hydroxyl group, nucleic acids having a hydroxyl group, and the like.

Specific examples of the alcohols exemplified as a compound having a hydroxyl group include aliphatic alcohols such as methanol, ethanol, isopropanol, butanol, isobutanol, tert-butanol, pentanol, isopentanol, hexanol, heptanol, octanol, nonanol, decanol, undecanol and dodecanol; aromatic alcohols such as benzyl alcohol, phenethyl alcohol, phenylpropyl alcohol, naphthol and tetrahydronaphthol; and polyalcohols such as glycerin and ethylene glycol.

Specific examples of the compound having a sugar structure include monosaccharides such as glucose, arabinose, fucose, galactose, mannose, xylose, fructose, lyxose, allose, arinose, ribose, talose, gulose, idose, altrose, sorbitol, mannitol, glucosamine, D-glucopyranose, D-galactopyranose, methyl α-D-glucopyranoside, β-mannopyranose, α-D-glucopyranosyl fluoride, β-D-glucopyranosyl fluoride, 1,2:5,6-di-O-isopropylidene-α-D-glucofuranoside, 1,2:5,6-di-O-isopropylidene-α-D-allofuranose, 1,2:3,4-di-O-isopropylidene-α-D-galactopyranose and 2,3:5,6-di-O-isopropylidene-α-D-mannofuranose; oligosaccharides such as maltose, isomaltose, turanose, gentiobiose, meribiose, pranteobiose, primererose, vicianose, nigerose, laminaribiose, rutinose, cellobiose, xylobiose, maltotriose, gentianose, melezitose, planteose, ketose, trehalose, sucrose, lactose, raffinose and xylotriose; polysaccharides such as amylose, ficoll, dextrin, starch, dextran, polydextrose, pullulan, cyclodextrin, glucomannoglycan, glucomannan, guar gum, gum arabic and glycosaminoglycan; and complex carbohydrates such as glycopeptide, glycoprotein, glycolipid and proteoglycan. A compound formed by protecting or deoxidizing a part of the hydroxyl groups of these compounds containing a sugar structure with an acyl group such as an acetyl group, an acetylethylcarbonyl group and a benzoyl group; an aralkyl group such as a benzyl group and a methoxybenzyl group; an isopropylidene group and a benzylidene group is also included in. Further, a compound containing a sugar structure that is bound with, for example, a resin or a polymer via a linker is also included in these compound having a sugar structure.

The steroid having a hydroxyl group includes, for example, β-cholestanol, cholesterol, digitonin, cortisone, estradiol, androsterone, stanolone, methyl hydroxycholate, stigma sterol and the like.

The alkaloid having a hydroxyl group includes, for example, tropin.

The amino acid having a hydroxyl group includes, for example, serine, threonine, tyrosine and the like. Further, an amino acid having a hydroxyl group of which the -NH group is protected with, for example, a tert-butoxycarbonyl group (Boc) is also included in the amino acid having a hydroxyl group related to the present invention.

The peptide having an amino acid residue having a hydroxyl group includes a peptide having an amino acid residue such as a serine residue, a threonine residue and a tyrosine residue. The number of the amino acid residues of the peptide is usually 2 to 10.

The terpenes having a hydroxyl group include, for example, menthol, borneol, isomenthol, menthol, isoborneol, tetrahydrolavandulol and the like.

The nucleic acids having a hydroxyl group include, for example, adenosine, cytidine, thymidine, uridine, 2',3'-O-isopropylidene uridine and the like. The nucleic acid having a hydroxyl group related to the present invention also includes a compound formed by protecting or deoxidizing part of the hydroxyl groups of these nucleic acids having a hydroxyl group with an acyl group such as an acetyl group, an acetylethylcarbonyl group and a benzoyl group; an aralkyl group such as a benzyl group and a methoxybenzyl group; an isopropylidene group and a benzylidene group. Further, the nucleic acids also include a nucleic acid bound with, for example, a resin or a polymer via a linker.

The compound represented by the general formula [2] may be commercially available or synthesized as appropriate by an ordinary method. A compound obtained by protecting a hydroxyl group of the above compound having the hydroxyl group with a benzyl group which may have a substituent, may be used.

In the deuteration method of the present invention, the heavy hydrogen source to be used includes, for example, a deuterated solvent, and specifically in the case where the heavy hydrogen is deuterium, for example, deuterated water (D₂O); deuterated alcohols such as deuterated methanol, deuterated ethanol, deuterated isopropanol, deuterated butanol, deuterated tert-butanol, deuterated pentanol, deuterated hexanol, deuterated heptanol, deuterated octanol, deuterated nonanol, deuterated decanol, deuterated undecanol and deuterated dodecanol; deuterated carboxylic acids such as deuterated formic acid, deuterated acetic acid, deuterated propionic acid, deuterated butyric acid, deuterated isobutyric acid, deuterated valeric acid, deuterated isovaleric acid and deuterated pivalic acid; and deuterated ketones such as deuterated acetone, deuterated methyl ethyl ketone, deuterated methyl isobutyl ketone, deuterated diethyl ketone, deuterated dipropyl ketone, deuterated diisopropyl ketone and deuterated dibutyl ketone; and organic solvents such as deuterated dimethyl sulfoxide. Among these, deuterated water and deuterated alcohols are preferable and specifically deuterated water and deuterated methanol are particularly preferable. Incidentally, deuterated water is preferable in view of ecology and operability. In the case where the heavy hydrogen is tritium, the deuterated solvent includes, for example, tritiated water (T₂O) and the like. These deuterated solvents may be used alone or in combination of 2 or more solvents as appropriate.

A deuterated solvent having at least one deuterated hydrogen atom in the molecule may be used. For example, a deuterated alcohol having the hydrogen atom of the hydroxyl group deuterated and a deuterated carboxylic acid having the hydrogen atom of the carboxyl group deuterated may be used. A deuterated solvent having all hydrogen atoms in the molecule deuterated is particularly preferable.

The more amount of a heavy hydrogen source is used, the more the deuteration of the present invention proceeds. From the economical standpoint, however, the amount of the heavy hydrogen atom contained in a heavy hydrogen source is preferably in the order of equimolar amount, 10 molar times, 20 molar times, 30 molar times and 40 molar times as the lower limit and in the order of 250 molar times and 150 molar times as the upper limit, relative to the amount of the deuteratable hydrogen atom of the benzyl position in the compound having a -O-benzyl group which may have a substituent.

In the deuteration method of the present invention, a reaction solvent may be used as necessary. A liquid that hardly dissolves a substrate can be used as a reaction solvent because the deuteration of the present invention may be carried out in a suspended reaction system. A liquid that dissolves easily a substrate, however, is preferable.

Specific examples of the reaction solvent to be used as necessary include ethers such as dimethyl ether, diethyl ether, diisopropyl ether, ethyl methyl ether, tert-butyl methyl ether, 1,2-dimethoxyethane, oxirane, 1,4-dioxane, dihydropyran and tetrahydrofuran; aliphatic hydrocarbons such as hexane, heptane, octane, nonane, decane and cyclohexane; alcohols such as methanol, ethanol, isopropanol, butanol, tert-butanol, pentanol, hexanol, heptanol, octanol, nonanol, decanol, undecanol and dodecanol; carboxylic acids such as formic acid, acetic acid, propionic acid, butyric acid, isobutyric acid, valeric acid, isovaleric acid and pivalic acid; ketones such as acetone, methyl ethyl ketone, methyl isobutyl ketone, diethyl ketone, dipropyl ketone, diisopropyl ketone and dibutyl ketone; dimethyl sulfoxide; and the like. Among these, for example, tetrahydrofuran, 1,4-dioxane and acetone are preferable and tetrahydrofuran and the like is particularly preferable. These reaction solvents may be used alone or in combination of 2 or more as appropriate.

When the deuteration method of the present invention is carried out, the gas phase of a deuteration reactor is preferably displaced with the hydrogen gas (either a light hydrogen gas or a heavy hydrogen gas can be used, the same hereinafter) that is necessary for the reaction. A mixed gas of a hydrogen gas and an inert gas such as nitrogen and argon may be used for the displacement.

The amount of the hydrogen gas to be used in the deuteration method of the present invention is preferably in the order of equimolar amount, 10 molar times, 20 molar times and 30 molar times as the lower limit, and in the order of 100 molar times and 40 molar times as the upper limit, relative to the amount of a compound having a -O-benzyl group which may have a substituent to be used as a reaction substrate.

Incidentally, a reactor is preferably kept at a sealed or nearly sealed condition to prevent hydrogen gas from dissipating in the deuteration method of the present invention. The nearly sealed condition includes a condition where a reaction substrate is continuously supplied to the reactor and a reaction product is continuously taken out like so-called continuous reaction.

A large amount of a palladium/carbon-ethylenediamine complex (hereinafter, may be abbreviated as Pd/C (en)) to be used as a catalyst would give not only an end product at a high deuteration ratio, but also a by-product at a high rate. Therefore, an optimum amount to give a high deuteration ratio and suppress formation of a by-product may be selected as appropriate according to the kind of a substrate or the kind of a -O-benzyl group which may have a substituent in the substrate. The amount of Pd/C (en) is usually a so-called catalyst amount and preferably in the order of 0.01 to 200% by weight, 0.01 to 150% by weight, 0.01 to 100% by weight, 0.01 to 50% by weight, 0.01 to 20% by weight, 0.1 to 20% by weight, 1 to 20% by weight and 10 to 20% by weight relative to a compound (substrate) having a -O-benzyl group which may have a substituent. The upper limit of the amount of a catalyst metal to be contained in the whole catalyst is preferably in the order of 20% by weight, 15% by weight, 10% by weight, 5% by weight and 2% by weight, whereas the lower limit is preferably in the order of 0.0005% by weight, 0.005% by weight, 0.05% by weight and 0.5 % by weight.

The palladium/carbon-ethylenediamine complex may be synthesized by a known method developed by the present inventors (J. Org. Chem. 1998, 63, 7990 - 7992) or obtained on the market as appropriate (for example, made by Wako Pure Chemical Industries, Ltd).

In the deuteration method of the present invention, an agent to be poisoned (generally also called catalyst poison) to the catalytic action of the palladium/carbon-ethylenediamine complex (Pd/C (en)) related to the present invention may be further added to the reaction system, which sometimes can suppress formation of by-products.

The agent to be poisoned includes sulfur-containing compounds such as dimethyl sulfoxide and diethyl sulfoxide; heavy metal ions such as a mercury ion, an arsenic ion, a lead ion, a bismuth ion and an antimony ion; halides such as sodium iodide and potassium iodide; amines such as trimethylamine, triethylamine, ethylenediamine, pyridine and morpholine; phosphines such as triphenylphosphine, diphenyl(tert-butyl)phosphinomethane, diphenyl(tert-butyl)phosphinoethane and diphenyl(tert-butyl)phosphinopropane; carbon monoxide, carbon dioxide and the like. Among these, amines are preferable and triethylamine is more preferable.

The amount of the agent to be poisoned to be used is usually 0 to 100 equivalents and preferably in the order of 0 to 50 equivalents, 0 to 20 equivalents, 0 to 10 equivalents and 0 to 5 equivalents relative to the amount of a catalyst metal contained in Pd/C (en) to be used as a catalyst.

The lower limit of the reaction temperature in the deuteration method of the present invention is usually 10°C and preferably in the order of 20°C, 40°C and 50°C, whereas the upper limit is usually 180°C and preferably in the order of 100°C and 60°C.

The lower limit of the reaction time in the deuteration method of the present invention is usually 30 minutes to 120 hours, preferably 1 to 108 hours, more preferably 3 to 96 hours and still more preferably 12 to 72 hours.

The deuteration method of the present invention is described in detail as follows.
That is, for example, 1 mole of a compound (substrate) having a -O-benzyl group which may have a substituent and 0.01 to 200% by weight of Pd/C (en) relative to the substrate are added to a deuterated solvent (for example, deuterated water) of which the amount is enough to contain the heavy hydrogen atoms 10 to 150 molar times relative to the deuteratable hydrogen atoms of the substrate. After the gas phase of the sealed reactor is displaced with hydrogen gas of 1 to 10 molar times relative to the substrate, the solution is subjected to reaction under stirring at room temperature to 50°C for about 12 to 72 hours to obtain a compound of which the benzyl position of the -O-benzyl group is deuterated.

When the obtained product is soluble in the deuterated solvent, the reaction solution is filtered to remove the catalyst after termination of the reaction. After the filtrate is concentrated, the product is isolated and subjected to structural analysis by ¹H-NMR, ²H-NMR and mass spectrum measurement. When the product is hardly soluble in the deuterated solvent, the product is isolated from the reaction solution and then subjected to structural analysis by ¹H-NMR, ²H-NMR and mass spectrum measurement.

Incidentally, when it is difficult to isolate the product from the reaction solution, the filtrate may be measured as it is with ¹H-NMR using a proper internal standard substance for structural analysis of the product. When the product is hardly soluble in the deuterated solvent, in order to isolate the product from the reaction solution, the product may be extracted from the reaction solution with, for example, an organic solvent in which the product is soluble and then purified by filtering off the catalyst according to a known purification method.

According to the deuteration method of the present invention, an objective compound, in which the benzyl position of a -O-benzyl group which may have a substituent is deuterated, can be obtained at a high deuteration ratio without suffering the hydrogenolysis of the -O-benzyl group in the compound.

The deuteration method of the present invention can be used in various fields of not only synthesis of a labeled compound but also investigation of reaction mechanism and the like, because the present method can selectively deuterate the benzyl position of a -O-benzyl group in a compound having the -O-benzyl group.

The present invention is described more specifically with reference to the following examples, however, to which the invention is not limited at all.

### EXAMPLES

The Pd/C (en) used in the following examples is commercially available (made by Wako Pure Chemical Industries, Ltd; Pd content 5% by weight, ethylenediamine content 5% by weight) and Pd/C is commercially available (made by Aldrich Inc.; Pd content 5%).
The deuteration ratio is determined as follows in the examples and comparative examples below.
That is, after termination of the reaction, the reaction solution was extracted with ether and the catalyst was filtered off. The filtrate was concentrated under reduced pressure and then subjected to structural analysis by ¹H-NMR, ²H-NMR and mass spectrum measurement to determine the deuteration ratio of the hydrogen atoms of the reaction substrate. Incidentally, in each table, each deuteration ratio shows the deuteration ratio of the hydrogen atom at the position indicated by the number noted in the structural formula of each reaction substrate.
The hydrogen atoms in an aromatic ring contained in a compound obtained in the following examples and comparative examples were not deuterated.

### Examples 1 to 3. Study on the deuteration ratios of the benzyl position of a -C-benzyl group and the benzyl position of a -O-benzyl group

0.5 mmol of compound (1) (substrate) represented by the formula below and 20% by weight of Pd/C (en) (palladium catalyst related to the present invention) relative to compound (1) were suspended in 1 mL of deuterated water. After the reaction system was deaerated, the reaction solution was contacted with 17 mL of light hydrogen gas using a balloon and subjected to reaction under stirring at the predetermined temperature and for predetermined time shown in Table 1. After the reaction, the obtained reaction solution was added with 10 mL of ether and the catalyst was filtered off with a membrane filter. The filtrate was extracted with 3 mL of water and 40 mL of ether. The obtained ether layer was washed with 20 mL of saturated salt water, dried and concentrated under reduced pressure. The residue was purified by silica gel chromatography (hexane/ether = 10/1) and subjected to structural analysis by ¹H-NMR and mass spectrum measurement and the objective deuterated compound (2) was obtained. The result is shown in Table 1.

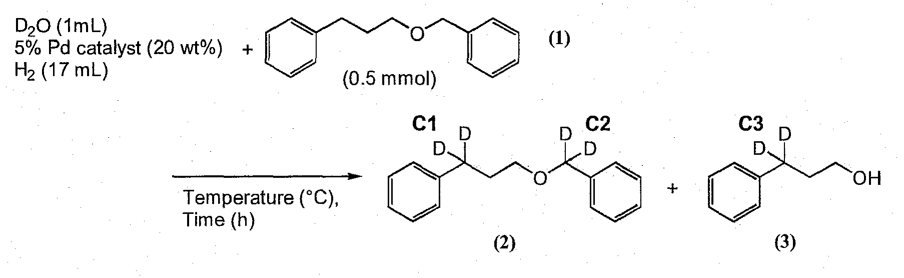

### Comparative Examples 1 and 2.

Similar reactions were carried out with similar operations to in Example 1 except that Pd/C was used as a palladium catalyst. The reaction conditions and results are also shown in Table 1.

**[Table 1]**

| | catalyst | reaction temp. (°C) | reaction time (h) | main product | | | by-product | |
|---|---|---|---|---|---|---|---|---|
| | | | | Deuteration ratio (%) | | yield (%) | Deuteration ratio (%) | yield (%) |
| | | | | C1 | C2 | | C3 | |
| Ex.1 | Pd/C(en) | r.t. | 24 | 4 | 33 | 95 | - | trace |
| Ex.2 | | 50 | 6 | 60 | 91 | 90 | - | trace |
| Ex.3 | | 50 | 24 | 95 | 97 | 60 | ND | ND |
| Com. Ex.1 | Pd/C | r.t. | 24 | 23 | 79 | 73 | 52 | 24 |
| Com. Ex.2 | | 50 | 6 | 84 | 92 | 36 | 91 | 60 |

| | | | | | | | | |
|---|---|---|---|---|---|---|---|---|
| * ND = Not-Detectable, Trace = trace amount, r.t. = room temperature Ex.; Example, Com.Ex.; Comparative Example | | | | | | | | |

As apparent from the results of Table 1, it can be understood that comparing the Pd/C (en) used in Examples 1 to 3, as a Pd catalyst related to the present invention and the Pd/C catalyst used in Comparative Examples 1 and 2, the deuteration ratio (C2) of the benzyl position of a -O-benzyl group is higher than the deuteration ratio (C1) of the benzyl position of a -C-benzyl group for both catalysts, and shows more efficient H-D exchange reaction.
In addition, as apparent from the comparison of Example 1 and Comparative Example 1 and the comparison of Example 2 and Comparative Example 2, it can be understood that although use of Pd/C (Comparative Examples 1 and 2) as catalyst gives higher catalyst activity than use of Pd/C (en) ( Examples 1 to 3), use of Pd/C forms a large amount of the by-product (3) due to dropping out of the benzyl position of a -O-benzyl group resulting in lower yield of the main product (2).
In other words, it can be understood that, when Pd/C (en) of the palladium catalyst related to the present invention is used for deuterating a compound having a -O-benzyl group, it is an effective catalyst that can selectively deuterate the benzyl position without dropping the -O-benzyl group.

### Examples 4 to 11. Deuteration reaction of the benzyl position of benzyl isoamyl ether

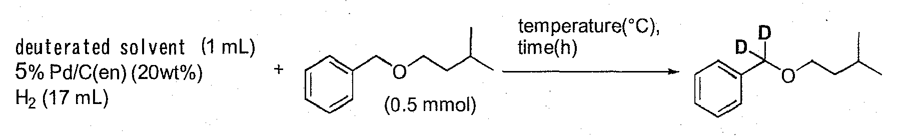

0.5 mmol of benzyl isoamyl ether (substrate) and 20% by weight of Pd/C (en) (palladium catalyst related to the present invention) relative to this ether were suspended in 1 mL of a deuterated solvent. After the reaction system was deaerated, the reaction solution was contacted with 17 mL of light hydrogen gas using a balloon and subjected to reaction at the predetermined temperature and for predetermined time shown in Table 2. After the reaction, the reaction solution was filtered with a membrane filter. The filtrate was subjected to structural analysis by ¹H-NMR and mass spectrum measurement as it was, and the objective deuterated compound was obtained. The results are shown in Table 2.

**[Table 2]**

| | deuterated solvent | reaction temp. (°C) | reaction time (h) | deuteration ratio (%) | yield (%) |
|---|---|---|---|---|---|
| Example 4 | D₂O | r.t. | 6 | 37 | 73 |
| Example 5 | D₂O | r.t. | 12 | 33 | 83 |
| Example 6 | D₂O | r.t. | 24 | 95 | 68 |
| Example 7 | D₂O | r.t. | 48 | 98 | 63 |
| Example 8 | D₂O | r.t. | 72 | 98 | 72 |
| Example 9 | D₂O | 50 | 12 | 98 | 47 |
| Example 10 | D₂O | 50 | 24 | 99 | 62 |
| Example 11 | CH₃OD | r.t. | 24 | 50 | 91 |

| | | | | | |
|---|---|---|---|---|---|
| * r.t. = room temperature | | | | | |

As apparent from the results of Examples 4 to 8, the deuteration method of the present invention can provide a compound with a nearly quantitative deuteration ratio in the reaction time of 24 hours even at room temperature. It can be also understood that the reaction is completed in 12 hours at 50°C (results of Examples 9 and 10). It is further understood that deuterated methanol also can provide the objective deuterated compound (results of Example 11).
In the deuteration of the benzyl position of a -O-benzyl group of the present invention, the reaction is completed in only 24 hours even at room temperature with the combination of Pd/C (en)-D₂O-H₂, while in the conventional method (Synlett 2002, 1149 - 1151) for deuterating the benzyl position with the combination of Pd/C-D₂O-H₂, the reaction takes 72 hours at room temperature to obtain a quantitative amount of a deuterated compound. It is apparent, therefore, that the deuteration method of the present invention is more efficient.

### Examples 12 to 19. Deuteration reaction of the benzyl position of benzyl isoamyl ether (use of a mixed solvent)

Similar operations to in Example 4 were carried out except that the reaction was at 50°C for 12 hours using the predetermined amounts of various deuterated solvents and the predetermined amounts of various reaction solvents as shown in Table 3 below, to deuterate the benzyl position of the objective compound. The results are also shown in Table 3.

### Examples 20 to 22. Deuteration reaction of the benzyl position of benzyl isoamyl ether (addition of an agent to be poisoned)

Similar operations to in Example 4 were carried out except that the reaction was at 50°C for 12 hours using the predetermined amounts of various deuterated solvents and the predetermined amounts of various reaction solvents as shown in Table 3 and 2 equivalents of various agents to be poisoned shown in Table 3 relative to the Pd amount in the palladium catalyst (Pd/C (en)) related to the present invention, to deuterate the benzyl position of the objective compound. The results are also shown in Table 3.

**[Table 3]**

| | deuterated solvent (mL) | reaction solvent (mL) | total amount of solvent (mL) | additive | deuteration ratio (%) | yield (%) |
|---|---|---|---|---|---|---|
| Exam 12 | D₂O (0.5) CH₃OD | - | 1 | - | 96 | 63 |
| Exam 13 | D₂O (0.5) | 1,4-dioxane (0.5) | 1 | - | 95 | 77 |
| Exam 14 | D₂O (0.5) | THF(0.5) | 1 | - | 96 | 74 |
| Exam 15 | D₂O (0.3) | THF(0.7) | 1 | - | 93 | 88 |
| Exam 16 | D₂O (0.1) | THF(0.9) | 1 | - | 60 | 93 |
| Exam 17 | D₂O (0.4) | THF(1.6) | 2 | - | 86 | 88 |
| Exam 18 | D₂O (0.6) | THF(1.4) | 2 | - | 96 | 78 |
| Exam 19 | CH₃OD (0.5) | THF(0.5) | 1 | - | 81 | 82 |
| Exam 20 | D₂O (1.0) | - | 1 | pyridine | 56 | 89 |
| Exam 21 | D₂O (1.0) | - | 1 | Et₃N | 97 | 87 |
| Exam 22 | D₂O (0.3) | THF(0.7) | 1 | Et₃N | 70 | 77 |

| | | | | | | |
|---|---|---|---|---|---|---|
| THF : tetrahydrofuran, Et₃N : triethylamine | | | | | | |

As apparent from the results of Example 9 in Table 2 and Examples 12 to 19 in Table 3, it can be understood that a mixed solvent of deuterated solvents or a mixed solvent of a deuterated solvent and a reaction solvent provides an objective compound in a higher yield.
It can be also understood that a mixed solvent of deuterated water and THF of a different mixing ratio also provides an objective compound having a high deuteration ratio in a high yield (results of Examples 14 to 18).
It can be further understood that deuteration yield is improved in the presence of an agent to be poisoned, particularly that both of the deuteration ratio and the yield are high in the presence of triethylamine (results of Examples 20 to 22).

### Examples 23 to 28. Deuteration reaction of benzyl 1,2,3,4-tetrahydro-1-naphthyl ether

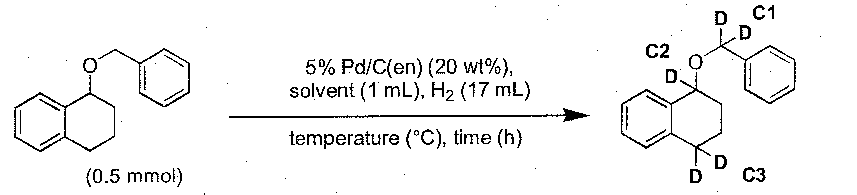

Similar operations to in Example 1 to 3 were carried out except that the reaction was carried out under the conditions shown in Table 4 using 0.5 mmol of benzyl 1,2,3,4-tetrahydro-1-naphthyl ether (substrate) and the predetermined amount shown in Table 4 of deuterated water or a mixed solvent of deuterated water and tetrahydrofuran (THF), to deuterate the benzyl position of the objective compound. The results are shown in Table 4.

**[Table 4]**

| | deuterated solvent (mL) | reaction solvent (mL) | reaction temp. (°C) | reaction time (h) | deuteration ratio (%) | | | yield (%) |
|---|---|---|---|---|---|---|---|---|
| | | | | | C1 | C2 | C3 | |
| Exam 23 | D₂O (1) | - | room temp. | 12 | 12^{(a)} | | 2 | 46 |
| Exam 24 | | | | 24 | 34^{(a)} | | 16 | 88 |
| Exam 25 | | | 50 | 12 | 96 | 77 | 83 | 73 |
| Exam 26 | | | | 24 | 98 | 71 | 76 | 59 |
| Exam 27 | D₂O (0.3 | THF (0.7) | 50 | 12 | 92 | 73 | 87 | 68 |
| Exam 28 | | | | 24 | 92 | 90 | 83 | 67 |

| | | | | | | | | |
|---|---|---|---|---|---|---|---|---|
| *(a): represents average value of deuteration ratios of C1 and C2 | | | | | | | | |

As apparent from the results of Examples 25 to 28, it can be understood that the deuteration of the benzyl position (C1) of a -O-benzyl group proceeds nearly quantitatively and provides a higher deuteration ratio than other benzyl positions (C2 and C3), which shows high activity of the Pd/C (en) catalyst for deuteration of the benzyl position of a -O-benzyl group.
It can be also understood that comparing the results of Examples 23 to 24 and Examples 25 to 26, the reaction at 50°C rather than room temperature can deuterate the hydrogen at the benzyl position (C1) more quantitatively.

### Examples 29 and 30. Deuteration reaction of the benzyl position of a -O-benzyl group using an amino acid

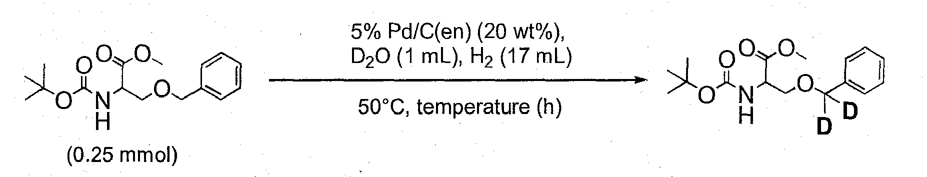

Similar operations to in Examples 1 to 3 were carried out except that 0.25 mmol of N-Boc-O-benzyl-L-serine-methyl ester (substrate) was used and that the reaction was carried out at 50°C, for the predetermine time shown in Table 5, to deuterate the benzyl position of the objective compound. The results are also shown in Table 5.
* BOC = tert-butoxycarbonyl group

**[Table 5]**

| | deuterated solvent (mL) | reaction temperature (°C) | reaction time (h) | deuteration ratio (%) | Yield (%) |
|---|---|---|---|---|---|
| Example 29 | D₂O (1) | 50 | 24 | 93 | 79 |
| Example 30 | | | 48 | 96 | 75 |

As apparent from the results of Examples 29 to 30, it can be understood that the benzyl position of a -O-benzyl group of a serine derivative (amino acid) shows a high deuteration ratio.

### Examples 31 to 50. Deuteration reaction of the benzyl position of a -O-benzyl group using saccharide

Similar operations to in Examples 1 to 3 were carried out except that 0.25 mmol of various substrates shown in Table 6 was used and that the predetermined amount of deuterated water or a mixed solvent of deuterated water and THF shown in Table 6 was used, to deuterate the hydrogen at the benzyl position of the objective compound under the conditions shown in Table 6. The results are also shown in Table 6.

**[Table 6]**

| | Substrate | deuterated solvent (mL) | reaction solvent (mL) | reaction temp. (°C) | reaction time (h) | C1 deuteration rate (%) | yield (%) |
|---|---|---|---|---|---|---|---|
| Example 31 | 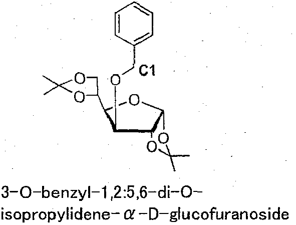 | D₂O (1) | - | 50 | 24 | 82 | 73 |
| Example 32 | | | | | 48 | 92 | 57 |
| Example 33 | | D₂O (0.3) | THF (0.7) | 50 | 48 | 74 | 85 |
| Example 34 | | | | | 72 | 92 | 77 |
| Example 35 | 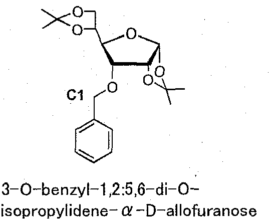 | D₂O (1) | - | 50 | 48 | 57 | 60 |
| Example 36 | | D₂O (0.3) | THF (0.7) | 50 | 24 | 50,83^{(b)} | 54 |
| Example 37 | | | | | 48 | 86 | 36 |
| Example 38 | | | | | 72 | 94 | 31 |
| Example 39 | 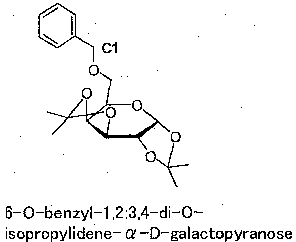 | D₂O (1) | - | r.t. | 72 | 42 | 81 |
| Example 40 | | | | 50 | 12 | 93 | 66 |
| Example 41 | | | | | 24 | 97 | 47 |
| Example 42 | | D₂O (0.3) | THF (0.7) | 50 | 12 | 76 | 93 |
| Example 43 | | | | | 24 | 91 | 81 |
| Example 44 | | | | | 48 | 94 | 67 |
| Example 45 | 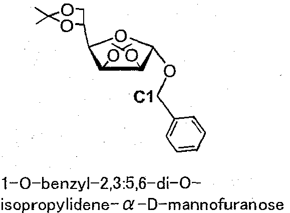 | D₂O (1) | - | 50 | 12 | 21,81^{(b)} | 46 |
| Example 46 | | | | | 24 | 33,86^{(b)} | 27 |
| Example 47 | | D₂O (0.3) | THF (0.7) | 50 | 12 | 76 | 93 |
| Example 48 | | | | | 24 | 91 | 81 |
| Example 49 | | | | | 48 | 94 | 67 |
| Example 50 | 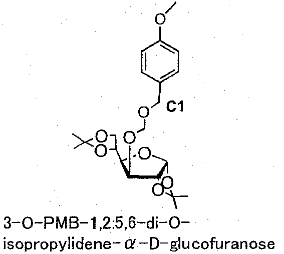 | D₂O (1) | - | 50 | 48 | 87 | 84 |

| | | | | | | | |
|---|---|---|---|---|---|---|---|
| * (b) shows each deuterationratio of 2 protons at C1 position * BOC=tert-butoxycarbonyl group * PMB=p-methoxybenzyl group * r.t. = room temperature | | | | | | | |

As apparent from the results of Examples 31 to 49, it can be understood that a high deuteration ratio is shown for the hydrogen at the benzyl position of a benzyl protecting group (benzyloxy group) of a hydroxyl group of the various substrates.
As apparent from the results of Example 50, it can be understood that a high deuteration ratio is also shown for the hydrogen at the benzyl position of a PMB protecting group (p-methoxybenzyloxy group) of a hydroxyl group of the substrate.
Further, an improved yield was seen when a mixed solvent of deuterated water and THF, rather than deuterated water alone, was used (results of Examples 32 versus 33, 40 versus 42, 41 versus 43, 45 versus 47, and 46 versus 48).

### Examples 51 to 53. Deuteration reaction of the benzyl position of a -O-benzyl group using saccharide

0.25 mmol of the substrate shown in Table 7 and the predetermined amount of 5% Pd/C (en) relative to the substrate were suspended in 1 mL of deuterated water. After the reaction system was deaerated, the reaction solution was contacted with 17 mL of light hydrogen gas using a balloon. Similar operations to in Example 1 were carried out except that the reaction solution was subjected to reaction at 50°C for 72 hours, to deuterate the benzyl position of the objective compound. The results are also shown in Table 7.

**[Table 7]**

| | substrate | 5%Pd/C(en) (wt%) | deuterated solvent (mL) | reaction temp. (°C) | reaction time (h) | C1 deuteration rate (%) | yield |
|---|---|---|---|---|---|---|---|
| Example 51 | 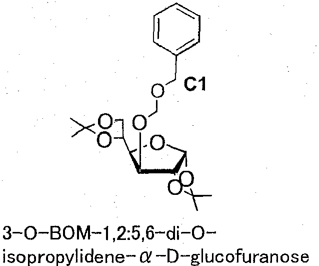 | 40 | D₂O (1) | 50 | 72 | 57 | 91 |
| Example 52 | | 80 | | | | 88 | 80 |
| Example 53 | | 100 | | | | 93 | 73 |

| | | | | | | | |
|---|---|---|---|---|---|---|---|
| 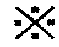 BOM=benzyloxymethyl group | | | | | | | |

As apparent from the results of Examples 51 to 53, a high deuteration ratio was obtained for deuteration of the benzyl position of a -O-benzyl group in the BOM protecting group (benzyloxymethyloxy group) of the hydroxyl group of saccharide. In particular, it can be also understood that use of the larger amount of the palladium catalyst (Pd/C (en) ) related to the present invention shows the higher deuteration ratio is obtained.

### Examples 54 to 56. Deuteration reaction of the benzyl position of a -O-benzyl group using steroids

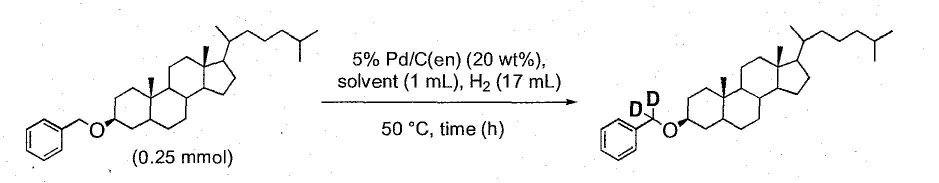

0.25 mmol of O-benzyl β-cholestanol (substrate) and 20% by weight of Pd/C (en) (palladium catalyst related to the present invention) relative to the substrate were suspended in the predetermined amounts of a heavy hydrogen and THF shown in Table 8. After the reaction system was deaerated, the reaction solution was contacted with 17 mL of light hydrogen gas using a balloon. Similar operations to in Example 1 were carried out except that the reaction solution was subjected to reaction at 50°C for the predetermined time shown in Table 6, to deuterate the benzyl position of the objective compound. The results are also shown in Table 8.

**[Table 8]**

| | deuterated solvent (mL) | reaction solvent (mL) | reaction time (h) | deuteration ratio (%) | yield (%) |
|---|---|---|---|---|---|
| Example 54 | D₂O (0.5) | THF (0.5) | 24 | 79 | 81 |
| Example 55 | D₂O (0.3) | THF (0.7) | 24 | 77 | 85 |
| Example 56 | | | 48 | 93 | 79 |

As apparent from the results of Examples 54 to 56, an improved deuteration ratio and yield was seen when the ratio of THF was increased.
It can be understood that when the reaction is carried out for 48 hours, the objective compound is obtained in a high deuteration ratio and a high yield (results of Example 56).

### Example 57. Deuteration reaction of the benzyl position of a -O-benzyl group using nucleic acids (substrate).

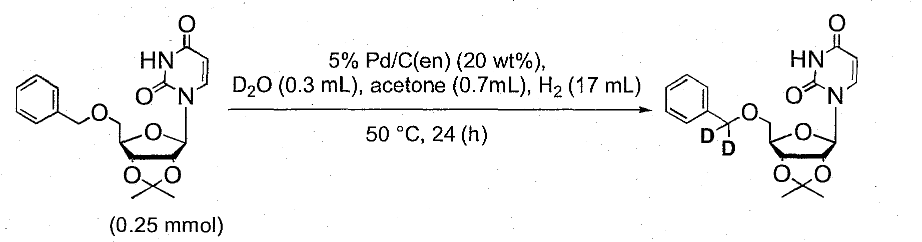

0.25 mmol of 5'-O-benzyl 2',3'-O-isopropylideneuridine (substrate) and 20% by weight of Pd/C (en) (palladium catalyst related to the present invention) relative to the substrate were suspended in 0.3 mL of heavy hydrogen and 0.7 mL of acetone. After the reaction system was deaerated, the reaction solution was contacted with 17 mL of light hydrogen gas using a balloon. After the reaction solution was subjected to reaction at 50°C for 24 hours, the reaction solution was filtered with a membrane filter. The filtrate was subjected to structural analysis by ¹H-NMR and mass spectrum measurement as it was,, to obtain the objective deuterated compound. The deuteration ratio was 35% and the yield was 78%.
As apparent from the results, it can be understood that the benzyl position of a -O-benzyl group in the sugar part of a nucleic acid can also be efficiently deuterated.
As apparent from the above obtained results, it can be understood that the H-D exchange reaction at the benzyl position of a -O-benzyl group in various substrates can be efficiently carried out by the deuteration method of the present invention.

## Claims

1. A method of deuterating the benzyl position of a -O-benzyl group of a compound having the -O-benzyl group which may have a substituent, comprising reacting the compound with a heavy hydrogen source in the coexistence of a palladium/carbon-ethylenediamine complex and hydrogen.

2. The method according to claim 1, wherein the compound is obtained by protecting a hydroxyl group of a compound having the hydroxyl group with a group represented by the general formula [1]: 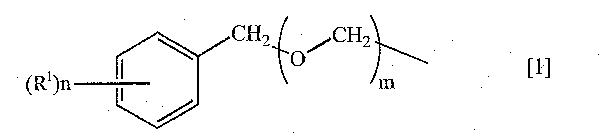 (wherein, n R¹s each independently indicate a light hydrogen atom, a heavy hydrogen atom, an alkyl group or an alkoxy group; n indicates 0 or an integer of 1 to 3; and m indicates 0 or 1).

3. The method according to claim 1, wherein the -O-benzyl group which may have a substituent is a group represented by the general formula [2]: 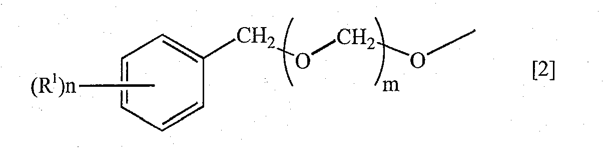 (wherein, n R¹s each independently indicate a light hydrogen atom, a heavy hydrogen atom, an alkyl group or an alkoxy group; n indicates 0 or an integer of 1 to 3; and m indicates 0 or 1).

4. The method according to claim 1, wherein the compound having a -O-benzyl group which may have a substituent is a compound represented by the general formula [3]: 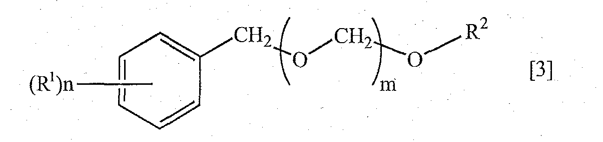 (wherein, n R¹s each independently indicate a light hydrogen atom, a heavy hydrogen atom, an alkyl group or an alkoxy group; R² is an alkyl group, aryl group, aralkyl group or heterocyclic group, which may have a substituent, or a monovalent group derived from a compound having a hydroxyl group, of which the hydrogen atom of the hydroxyl group is substituted with a binding arm; n indicates 0 or an integer of 1 to 3; and m indicates 0 or 1).

5. The method according to claim 1, wherein the heavy hydrogen source is a deuterated solvent.

6. The method according to claim 5, wherein the deuterated solvent is deuterated water (D₂O).
